# EUROPEAN PATENT APPLICATION

(11) **EP 1 774 973 A1**
(43) Date of publication of application: **18.04.2007**
(21) Application number: 05077579.0
(22) Date of filing: 11.11.2005
(51) Int. Cl.: A61K 35/20, A61P 3/10

(54) **Leucine rich composition**

(30) Priority: 12.10.2005 EP 05077339
(71) Applicant: Nutricia N.V., 2712 HM Zoetermeer (NL)
(72) Inventor: Peters, Johannes Adrianus Cornelis, 3512 TX Utrecht (NL); Hageman, Robert Johan Joseph, 6705 CT Wageningen (NL)
(74) Representative: Winckels, Johannes Hubertus F.

(57) **Abstract**

The invention relates to the use of a composition comprising proteinaceous matter, said providing at least 24.0 % of the energetic value of the composition (en%) and at least 12 wt. % based on proteinaceous matter leucine in the manufacture of a medicament for the treatment of insulin resistance and to a composition suitable for administration to a mammal comprising a protein fraction, a carbohydrate fraction and a lipid fraction, wherein the protein fraction provides at least 24.0 % (en%), the carbohydrate provides up to 46 en%, the lipid fraction provides up to 30 en% and wherein at least 12 wt. %, based on proteinaceous matter, of leucine.

## Description

The invention relates to the treatment of insulin resistance and to a composition suitable to such purpose or a related purpose.

Insulin resistance (IR) is the loss of sensitivity of the body towards insulin.

IR may be the result of trauma or an illness. For instance the occurrence of severe burns has been reported to cause IR. Surgery may be another cause. Further, IR has been observed as in patients suffering from various diseases, e.g. in terminally ill cancer patients, in AIDS patients, patients suffering from chronic pulmonary diseases or from severe inflammation. IR may also occur as a consequence of a metabolic syndrome, diabetes or during obesity. IR may also develop as a result of aging.

A reduced effect of insulin has consequences for the glucose metabolism in muscles, the liver and the pancreas. The inventors have come to the conclusion that insulin also plays a considerable role in the protein metabolism, in particular in muscle anabolism or the avoidance of muscle catabolism. In particular in patients suffering from a severe disease, e.g. AIDS, chronic obstrusive pulmonary disease or cancer, IR may give rise to a worse prognosis with respect to the chance of survival or life expectancy. Also it may be detrimental to the quality of life.

The inventors further have concluded that, in particular in patients suffering from a metabolic disorder, the disturbed protein metabolism as a consequence of IR may result in a reduction in the lean body mass. This may be worsened as a result of too little exercise. For instance, immobilised patients may be severely catabolic with respect to muscle mass.

It is an object of the invention to provide a preparation suitable for treating IR or a symptom associated with IR. In particular it is an object to provide a pharmaceutical composition or a food composition for such purpose.

Preferably, the composition should be suitable to treat a catabolism, such as muscle catabolism, in a patient suffering from IR.

Preferably, the organoleptic properties should be such that the consumption is generally appreciated as pleasant.

Preferably, the composition should pass the stomach easily.

Preferably, the digestible components of the composition should become readily available upon intake of the product.

In an aspect of the invention, it is a further object to increase the insulin sensitivity of a subject and preferably at the same time reducing muscle catabolism.

In a further aspect, the invention aims to improve the prognosis and/or the quality of life of the patient treated with the composition.

It has now been found that it is possible to treat a subject with IR with a specific composition comprising proteinaceous matter.

Accordingly, the present invention relates to the use of a composition comprising proteinaceous matter, said proteinaceous matter providing at least 24.0 % of the energetic value of the composition (en%), and at least 12 wt. % based on proteinaceous matter leucine in the manufacture of a medicament for the treatment of insulin resistance, in particular for the treatment of a catabolic condition in a patient suffering from insulin resistance. The catabolic condition preferably is muscle catabolism.

The invention further relates to a composition suitable for administration to a mammal comprising a protein fraction, a carbohydrate fraction and a lipid fraction, wherein the protein fraction provides at least 24.0 % (en%), the carbohydrate provides up to 46 en%, the lipid fraction provides up to 30 en% and wherein at least 12 wt. %, based on proteinaceous matter is leucine.

The invention allows reduction of at least one of the symptoms associated with the insulin resistance. In particular, protein catabolism, more in particular muscle catabolism may be reduced. Besides protein anabolism, such as muscle anabolism, may be increased. For instance, a positive net protein balance is feasible, for at least one hour after administering a medicament/composition as defined in the present invention. In an embodiment this is feasible for a subject suffering from an undesired loss of body weight (over 5 % in 3 months) or in subjects running a serious risk to be confronted with such loss; a subject confronted with a trauma; a subject having undergone surgery or a medical treatment with large impact (such as chemo- or radiotherapy) or subjects suffering from a severe disease; chronically immobilized subjects; wherein said subjects have developed IR.

The invention provides in a better prognosis in terms of extended life-expectancy and/or a better quality of life. Factors improving the quality of life are in particular less fatigue, more stamina, less complications such as viral and/or bacterial infections (in particular in mouth, throat, intestine, wounds and lungs), reduced loss of visual capability and/or hearing, better general condition and less periods of feeling depressed.

Subjects suffering from IR tend to have an increased plasma glucose level after fasting (e.g. in the morning after a night of sleep). IR is in particular determinable by the HOMA ratio glucose/insulin. HOMA modelling is described by Wallace et al. in Diabetes Care, Volume 27, Number 6, June 2004 p 1487-1495.

Proteinaceous matter are moieties formed from amino acids. The term amino acids as used herein includes amino acid residues (*e.g*. in peptides). In particular proteinaceous matter includes free amino acids, amino acid salts, the amino acid residues bound to conjugating molecules and peptides. The peptides include oligopeptides as well as proteins and other polypeptides. Likewise, when reference is made to a specific amino acid, *e.g*. leucine, this is meant to include the specific amino acid (residues) present as a salt, in a bound form, as well as the free specific amino acid.

The energetic value of a compound (en %) is based on the energy provided by the digestible part (in particular in a human or other mammal) of the compound. In particular the energetic value is based on the contribution of proteinaceous matter, lipids and digestible carbohydrates, using the following calculation factors: 4 kcal/g for digestible carbohydrates and proteinaceous matter and 9 kcal/g for lipids.

Slowly digestible carbohydrates, are digested less fast than maltodextrose, maltose and glucose. In particular a carbohydrate is considered slowly digestible in case more than 10 % of the sugars is released within 20 and 120 min in an analysis setting using standard digestive enzymes, as determinable by the Enquist method.

Carbohydrates that are digested rapidly include maltodextrose, maltose or glucose (quickly digestible carbohydrates). In particular within 20 min more than 90 % of quickly carbohydrates are digested in accordance with the Enquist method.

Low glycemic disaccharides are in particular disaccharides having a glycemic index number < 60 (glucose=100).

In an embodiment at least part of the proteinaceous matter is present in the form of free amino acids, a salt thereof or as a conjugate with a conjugating molecule other than a protein which conjugate is capable of being split in the free amino acid (or salt thereof) and the conjugating compound under the influence of a bile constituent and/or a pancreas excretia in duodenum and/or the ileum. In an embodiment, the amount of amino acid in such form, in particular in the form of a salt or the free form is up to 15 wt. % based on the proteinaceous matter, preferably 0.5-14 wt. %.

Leucine is preferably for 35-100 wt. %, more preferably for 40-80 wt. % based on the total weight of leucine, in the form of a peptide (oligopeptide, polypeptide, protein), preferably in the form of polypeptides and/or (intact) proteins. Thus, the uptake of leucine by the body is considered to be slower, which is advantageous in view of treating IR, in particular of treating (muscle) catabolism in a patient suffering from IR.

In a preferred embodiment, the composition (used) in accordance with the invention further comprises a sustained release preparation effective to release an amino acid in the duodenum and/or the ileum, said preparation comprising at least one component selected from the group consisting of amino acids in the form of a free acid, amino acids in the form of a salt and amino acids in the form of a conjugate with a conjugating compound other than a protein which conjugate is capable of being split in the free amino acid (or salt thereof) and the conjugating compound under the influence of a bile constituent and/or a pancreas excrements in duodenum and/or the ileum.

The inventors have realised that thus these free amino acids respectively salts thereof can become available for absorption in the duodenum or ileum at essentially the same time as or later than the amino acids provided in the form of polypeptides (including proteins).In particular for essential amino acids, this is considered to be advantageous in view of treating IR, in particular with respect to treating protein (muscle) catabolism in IR-patients.

The amino acid in the sustained release form is preferably suspended in a fluid, semi-fluid or solid product.

The sustained release preparation can be made based upon conventional techniques. The amino acid(s) may be coated with a pH sensitive material that dissolves at the pH existing in the duodenum/ileum (about pH 7) but not in the stomach (strongly acidic). Such coatings are generally known in the art. Examples of conjugating molecules are molecules forming specific peptides with the amino acid that are not split by pepsin, or at least not efficiently split under physiological conditions. Examples are choline, betain, dimethylglycine and sarcosine. Other suitable conjugating molecules include phospholipids, lyso-phospholipids and glycerol.

Amino acids that are preferably present in the sustained release preparation are preferably selected from leucine and other essential amino acids, in particular methionine, arginine, tryptophan, phenylalanine and lysine, of which leucine is especially preferred.

In a composition (used) according to the invention the wt. ratio Leucine/(valine+isoleucine) is generally 1.0 or more, preferably 1.05 or more.

It is preferred that the total amount of isoleucine (wt.) in the product exceeds the total amount of valine (wt). In case intact protein, which usually is present in the composition, does not provide a weight ratio of isoleucine to valine above one, one or more additional compounds can be included to influence the isoleucine/valine ratio, such as peptides that are rich in isoleucine and relatively poor in valine, or other compounds that are relatively rich in isoleucine or even isoleucine (preferably L-isoleucine) as base or salt.

In the total product the content of essential amino acids usually is at least 49 wt. %, preferably 49-80 wt. %, more preferably 52-70 wt. % of the proteinaceous matter is formed by essential amino acids.

The lysine content usually is 7.0-15 g/100g proteinaceous matter, preferably 7.5 to 14 g/100 g proteinaceous matter.

In an embodiment the composition (used) according to the invention comprises a whey protein fraction, in particular at least 10 wt. % based upon the proteinaceous matter, preferably at least 15 wt. % based upon the proteinaceous matter. Usually, the whey protein fraction is 50 wt. % or less based on proteinaceous matter. In particular in case of a fluid composition the concentration preferably does not exceed 35 wt. % based upon the proteinaceous matter. This is advantageous with respect to avoiding the risk of gelation during storage.

The presence of a whey protein may offer a number of advantages. The whey protein shows an advantageous release behaviour both in terms of release rate and the tendency to make the essential amino acids available for uptake by the body, essentially at the same time. This allows the degree of muscle anabolism as a result of a treatment with the composition.

Such effect may be further enhanced by (slightly) hydrolysing at least part of the whey protein, usually to the extent that up to 20 % of the protein is hydrolysed to free amino acids, preferably to the extent that up to 10 % of the protein is hydrolysed to free amino acids.

For said enhanced effect usually 50 wt. % of the whey protein or less is (slightly) hydrolysed, in particular 10-50 wt. %.

If desired the free amino acid or part thereof may be removed from the hydrolysate. Suitable techniques are known, e.g. filtration, chromatography or absorption.

As the source for whey protein(s) preferably a whey fraction is chosen comprising less that 20wt% casein glycomacropeptide (GMP), more preferably less than 10 g/100 g protein.

The beta- lactoglobulin content preferably is larger than 40wt%, more preferably 46-80 wt%.

Casein may be present as proteinacous matter. When used as intact protein, the casein preferably comprises a high concentration of beta casein, in particular more than 36 g/100 g casein, more in particular 38-70 g/100 g casein.

In a composition (used) in accordance with the invention, the lipid content is preferably up to 30 en%, more preferably 5-30 en%, even more preferably 5-29.0 en%.

For a high quality composition it is preferred that it provides sufficient essential fatty acids. For that purpose, the ratio n-3 to n-6 poly unsaturated fatty acids is preferably at least 0.2:1 to 10:1, more preferably in the range of 0.4:1 to 3:1. In a particular embodiment the ratio is at least 1.0.

The lipids, in particular the n-3 respectively n-6 poly unsaturated fatty acids typically include C18 to C26 fatty acids. Preferred examples thereof are alpha linolenic acid, eicosapentaenoic acid, docosahexaenoic acid, docosapentaenoic acid, linoleic acid and arachidonic acid.

The n-3 poly unsaturated fatty acids preferably are at least predominantly selected from the group consisting of alpha linolenic acid, eicosapentaenoic acid and docohexaenoic acid. The n-6 poly unsaturated fatty acids preferably are at least predominantly are selected from the group consisting of linoleic acid and arachidonic acid.

The carbohydrate fraction preferably provides a relatively low amount of the energetic value of the composition, usually 46 en % or less. In view of treating IR this is considered advantageous, because the postprandial amino acid response (the amino acid concentration measurable in blood after intake of the composition) may be increased, which in particular helps to reduce a symptom of IR, such as muscle catabolism as a result of IR.

In view of the above, the carbohydrate fraction content preferably provides 4 to 45.0 en%, more preferably, 8 to 44.0 en%, even more preferably 10-30 en% of the total composition.

The inventors have further contemplated that it is desirable to accomplish a fairly modest insulin response after intake of the composition. The choice of amino acids (in particular free amino acids or salts thereof) present in the composition plays a role. In particular arginine and lysine have a stimulating effect on the release of insulin and are therefore preferably not added (in free form or as a salt thereof) or in a relatively low content, in order to moderate the immune response. Especially the composition of the carbohydrate fraction may be chosen to achieve a favourable carbohydrate uptake, and accordingly a desirable insulin release after intake. Accordingly, in particular a composition meeting one or more of the following criteria with respect to the carbohydrate content are considered to be advantageous.

In an embodiment less than 75 wt. % of the carbohydrates is formed by the sum of the sucrose and the maltodextrin content.

In an embodiment at least 40 wt.% based on the total weight of the carbohydrates is formed by slowly digestible carbohydrates, i.e. in particular carbohydrates which are digested less fast than maltodextrose, maltose and glucose

In an embodiment a composition (used) according to the invention comprises less than 60 wt. %, preferably 20-50 wt. % based on the total weight of the carbohydrates of quickly digestible carbohydrates, in particular of maltodextrose, maltose, glucose and other carbohydrates which are digested at least as fast.

In an embodiment more than 20 wt. % based on the total weight of the carbohydrates is formed by at least one disaccharide, preferably 22-60 wt. %. In particular in such an embodiment, the disaccharide is preferably selected from the group consisting of sucrose, trehalose, palatinose, lactose and other low glycemic disaccharides, more preferably from trehalose and palatinose.

In an embodiment at least one monosaccharide other than glucose is present. Preferably said monosaccharide is selected from the group consisting of galactose, mannose and ribose. Preferably the total amount of said monosaccharide(s) is 0.5-30 wt. %, more preferably 5-25 wt. % based on the total weight of the carbohydrates.

In particular, the presence of ribose is advantageous, preferably in combination with (endogenous) folic acid, to increase the protein synthesis. It is contemplated that the combination of these two compounds allows an increase in the production of guanosine triphosphate in the subject, resulting in an increase of the protein synthesis via stimulation of eukaryotic initiation factor 2B, especially in malnourished patients.

The folic acid may be provided in one or more of the following forms: free folic acid, folinic acid, formylated folic acid, methylated folic acid, preferably in a reduced form or as a mono- or polyglutamate conjugated derivative.

When present, the folic acid content is usually at least 95 µg per 100 kcal carbohydrates, preferably 110-400 µg per 100 kcal carbohydrates, more preferably 125-300 µg per 100 kcal carbohydrates.

Besides folic acid one or more other additional components such as at least one component selected from the group consisting of minerals, trace elements and vitamins, preferably selected from the group consisting of vitamin A, vitamin C, vitamin D, vitamin E, vitamin B6, vitamin B1, vitamin B2, biotin, lipoic acid, vitamin B12 and zinc.

Such components may be present in a concentration up to the daily recommended dose per daily serving.

Zinc is preferably present in a concentration of at least 2.8 mg per 100 kcal carbohydrates, more preferably of 5.6-20 mg per 100 kcal carbohydrates, even more preferably of 6-15 mg per 100 kcal carbohydrates.

In an advantageous embodiment, a composition according to the invention is administered in a drug regimen. In particular the composition can be used as adjuvant of a drug, such as a drug selected from the group consisting of anti-cancer drugs, anti-retroviral drugs,antihypertensives, anti-thrombotics, anti-depressants and anti-diabetic drugs. In particular, it is advantageous to use the product with metformin or another anti-diabetic drug. These drugs in particular are considered to be stable in a composition according the invention and to be very effective. Said drug may be present in the composition according to the invention or be administered separately.

The total energetic value of a composition (used) in accordance with the invention may be chosen within wide limits. Usually it is at least 0.3, preferably at least 0.4 even more preferably at least 0.5 kcal/ml. In generally the value is up to 2.0, preferably up to 1.7, in particular up to 0.9 kcal/ml. Factors that play a role in determining a desirable energetic value include the ease of achieving a higher en% proteinaceous matter on the one hand and a fast emptying of the stomach (increasing anabolic response) on the other hand.

In this respect a product with an energetic value in the range of 0.5-0.9 kcal/ml has been found particularly advantageous. Further such product has a high tolerance level by the consumer.

The invention further relates to a method for treating insulin resistance, in particular a catabolic symptom in a patient suffering from IR, comprising administering a composition comprising a protein fraction providing at least 24.0 % of the energetic value of the composition (en%) and at least 12 wt. % based on the proteinaceous matter leucine to a subject.

The invention further relates to a method for treating muscle catabolism as a result of insulin resistance, comprising administering a composition comprising a protein fraction providing at least 24.0 % of the energetic value of the composition (en%) and at least 12 wt. % based on the proteinaceous matter leucine to a subject.

The invention further relates to the use of a composition as defined herein for improving the quality of life, in particular for an immobilised person and/or an elderly person.

The subject preferably is a human.

The method/composition of the invention may in particular be used for treating a subject suffering from at least one disorder selected from the group consisting of metabolic syndrome, diabetes, obesity, cancers, AIDS, chronic pulmonary diseases and severe inflammation disorders.

The composition is preferably in the form of a fluid.

A serving size of a composition (used) in accordance with the invention preferably is 50-500 g, more preferably 75-400 g, even more preferably 100-250 g. The composition may be administered once a day or several times a day.

The composition may be administered enterally.

## Claims

1. Use of a composition comprising proteinaceous matter, said proteinaceous matter providing at least 24.0 % of the energetic value of the composition (en%) and at least 12 wt. % based on proteinaceous matter leucine in the manufacture of a medicament for the treatment of insulin resistance.

2. Composition suitable for administration to a mammal comprising a protein fraction, a carbohydrate fraction and a lipid fraction, wherein the protein fraction provides at least 24.0 % (en%), the carbohydrate provides up to 46 en%, the lipid fraction provides up to 30 en% and wherein at least 12 wt. %, based on proteinaceous matter, is leucine.

3. Composition according to claim 2, wherein the composition further comprises a sustained release preparation effective to release an amino acid in the duodenum and/or the ileum, said preparation comprising at least one component selected from the group consisting of amino acids in the form of a free acid, amino acids in the form of a salt and amino acids in the form of a conjugate with a conjugating compound other than a protein which conjugate is capable of being split in the free amino acid (or salt thereof) and the conjugating compound under the influence of a bile constituent and/or a pancreas excretia in duodenum and/or the ileum.

4. Composition according to claim 3, wherein the preparation comprises at least one amino acid selected from the group consisting of leucine, methionine, arginine, tryptophane, phenylalanine and lysine, preferably the group consisting of leucine, methionine, tryptophane and phenylalanine, more preferably leucine.

5. Composition according to any one of the claims 2-4, wherein the protein fraction content provides 24.0 to 70 en%, preferably, 25.0 to 50 en%, of the total composition.

6. Composition according to any one of the claims 2-5, wherein the protein fraction comprises at least one protein selected from whey proteins, soy proteins and caseins, preferably at least one whey protein, more preferably a beta-lactoglobuline.

7. Composition according to claim 6 wherein the total amount of whey protein is at least 10 wt. % of the total weight of the proteinaceous matter, preferably 15-35 wt. % of the total weight of the proteinaceous matter.

8. Composition according to any one of the claims 2-7, wherein the lipid fraction content provides 5-29 en% of the total composition

9. Composition according to any one of the claims 2-8, wherein the lipid fraction comprises at least one n-3 poly unsaturated fatty acid and at least one n-6 poly unsaturated fatty acid, the ratio (wt to wt) of n-3 poly unsaturated fatty acid(s) to n-6 poly unsaturated fatty acids preferably being in the range of 0.2:1 to 10:1, more preferably in the range of 0.4:1 to 3:1.

10. Composition according to claim 8 or 9 comprising at least one polyunsaturated fatty acid having 18-26 carbon acids, preferably at least one polyunsaturated fatty acid selected from the group consisting of alpha linolenic acid, eicosapentaenoic acid, docosahexaenoic acid, linoleic acid and arachidonic acid.

11. Composition according any one of the claims 2-10, wherein the carbohydrate fraction content provides 4 to 45 en%, preferably, 8 to 44 en%, more preferably 10-30 en% of the total composition.

12. Composition according to any one of the claims wherein at least 40 wt.% based on the total weight of the carbohydrates is formed by slowly digestible carbohydrates, *i.e.* in particular carbohydrates which are digested less fast than maltodextrose, maltose and glucose

13. Composition according to any one of the claims 2-12 comprising 20-50 wt. % based on the total weight of the carbohydrates of quickly digestible carbohydrates, in particular of maltodextrose, maltose, glucose and other carbohydrates which are digested at least as fast.

14. Composition according to any one of the claims 2-13, comprising more than 20 wt. % based on the total weight of the carbohydrates, of at least one disaccharide, preferably 22-60 wt. %

15. Composition according to claim 14, wherein the disaccharide is selected from the group consisting of sucrose, trehalose, palatinose, lactose and other low glycemic disaccharides, preferably from trehalose and palatinose.

16. Composition according to any one of the claims 2-15, comprising at least one monosaccharide other than glucose, such as at least one monosaccharide selected from the group consisting of galactose, mannose and ribose.

17. Composition according to claim 16 wherein the total amount of said monosaccharide(s) is 0.5-30 wt. %, preferably 5-25 wt. % based on the total weight of the carbohydrates.

18. Composition according to any one of the claim 2-17 wherein at least 49 wt. %, preferably 49-80 wt. %, more preferably 52-70 wt. % of the proteinaceous matter is formed by essential amino acids.

19. Composition according to any one of the claim 2-18, wherein the energetic value of the composition is in the range of 0.3-2.0, preferably 0.4-1.7 kcal/ml, more preferably 0.4-1.2 kcal/ml, even more preferably 0.5-0.9 kcal/ml.

20. Composition according to any one of the claim 2-19, wherein the wt. ratio Leucine/(valine+isoleucine) is 1.0 or more, preferably 1.05 or more.

21. Composition according to any one of the claim 2-20, wherein the lysine content is 7.0-15 g/100g proteinaceous matter, preferably 7.5 to 14 g/100 g proteinaceous matter.

22. Composition according to any one of the claims 2-21 comprising at least one component selected from the group consisting of minerals, trace elements and vitamins, preferably selected from the group consisting of vitamin A, vitamin C, vitamin D, vitamin E, vitamin B6, vitamin B1, vitamin B2, biotin, lipoic acid, vitamin B12 and zinc.

23. Composition according to claim 22, comprising folic acid, preferably in the form of free folic acid, folinic acid, formylated folic acid, methylated folic acid, more preferably in a reduced form or as a mono- or polyglutamate conjugated derivative.

24. Composition according to claim 23 wherein the folic acid content is at least 95 µg per 100 kcal carbohydrates, preferably 110-400 µg per 100 kcal carbohydrates, more preferably 125-300 µg per 100 kcal carbohydrates.

25. Composition according to any one of the claims 22-24, comprising zinc, preferably in a concentration of at least 2.8 mg per 100 kcal carbohydrates, more preferably of 5.6-20 mg per 100 kcal carbohydrates, even more preferably of 6-15 mg per 100 kcal carbohydrates.

26. Method for treating insulin resistance, comprising administering a composition comprising a protein fraction providing at least 24.0 % of the energetic value of the composition (en%) and at least 12 wt. % based on the proteinaceous matter leucine to a subject.

27. Method according to claim 26, wherein the treatment involves treating muscle catabolism as a result of insulin resistance.

28. Method for treating muscle catabolism as a result of insulin resistance, comprising administering a composition comprising a protein fraction providing at least 24.0 % of the energetic value of the composition (en%) and at least 12 wt. % based on the proteinaceous matter leucine to a subject.

29. Method according to claim 26, 27 or 28, wherein the composition is a composition according to any one of the claims 2-25.

30. Method according to any one of the claims 26-29, wherein the subject is a human.

31. Method according to any one of the claims 26-30, for treating a subject suffering from at least one disorder selected from the group consisting of metabolic syndromes, in particular diabetes and/or obesitas; cancers; AIDS; chronic pulmonary diseases and severe inflammation disorders.

32. Method according to any one of the claims 26-31, wherein the composition is administered in the form of a fluid.

33. Method according to any one of the claims 26-32, wherein the composition is administered in a serving size of 50-500 g, preferably 75-400 g, more preferably 100-250 g

34. Method according to any one of the claims 26-33, wherein the composition is administered enterally.

35. Use of a composition as defined in any one of the claims 1-25 for improving the quality of life, in particular of an immobilised person and/or an elderly person.
